# EUROPEAN PATENT SPECIFICATION

(11) **EP 3 167 837 B1**
(45) Date of publication and mention of the grant of the patent: **02.01.2019**
(21) Application number: 16201737.0
(22) Date of filing: 25.11.2013
(51) Int. Cl.: A61B 18/18

(54) **ECOGENIC COOLED MICROWAVE ABLATION ANTENNA**
ÖKOGENGEKÜHLTE MIKROWELLENABLATIONSANTENNE
ANTENNE REFROIDIE D'ABLATION ÉCOGÉNIQUE À MICRO-ONDES

(30) Priority: 03.04.2013 US 201313856363
(43) Date of publication of application: 17.05.2017
(62) Divisional of application: 13194230.2
(73) Proprietor: Covidien LP, Mansfield, MA 02048 (US)
(72) Inventor: PETERSON, Darion R, Longmont, CO 80503 (US); CONNOLLY, Joseph M, Central, SC 29630 (US); DECARLO, Arnold V, Frederick, CO 80530 (US)
(74) Representative: Pratt, Richard Wilson

(56) References cited:
- US-A- 5 759 154

## Description

### BACKGROUND

### Technical Field

The present disclosure relates generally to medical/surgical ablation assemblies and methods of their use. More particularly, the present disclosure relates to an ecogenic cooled microwave ablation system and antenna assemblies configured for direct insertion into tissue for diagnosis and treatment of the tissue and methods of using the same.

### Background of Related Art

In the treatment of diseases such as cancer, certain types of cancer cells have been found to denature at elevated temperatures (which are slightly lower than temperatures normally injurious to healthy cells). These types of treatments, known generally as hyperthermia therapy, typically utilize electromagnetic radiation to heat diseased cells to temperatures above 41°C while maintaining adjacent healthy cells at lower temperatures where irreversible cell destruction will not occur. Other procedures utilizing electromagnetic radiation to heat tissue also include ablation and coagulation of the tissue. Such microwave ablation procedures, e.g., such as those performed for menorrhagia, are typically done to ablate and coagulate the targeted tissue to denature or kill it. Many procedures and types of devices utilizing electromagnetic radiation therapy are known in the art. Such microwave therapy is typically used in the treatment of tissue and organs such as the prostate, heart, and liver.

One procedure generally involves the treatment of tissue (e.g., a tumor) underlying the skin via the use of a percutaneously inserted microwave energy delivery device. The microwave energy delivery device penetrates the skin and is positioned relative to the target tissue, however, the effectiveness of such a procedure is often determined by the precision with which the microwave energy delivery device is positioned. Thus, the placement of the microwave energy delivery device requires a great deal of control. Reference is made to document US5759154.

### SUMMARY

The invention is defined in the appended set of claims. Disclosed is a surface treatment formed on the surface of a surgical device. The surface treatment includes an indentation extending into a surface of the surgical device. The indentation includes a first surface forming a first plane, a second surface forming a second plane, and a third surface forming a third plane. The first, second and third planes are substantially perpendicular to each other. The surface treatment is visible to an ultrasonic imaging system and the surface treatment improves visibility of the surgical device by the ultrasonic imaging system. The angle between the first plane and the surface of the surgical device may be between 10 and 20 degrees or the angle between the first plane and the surface of the surgical device may be between 70 and 80 degrees.

In another aspect, a surface treatment formed on the surface of a surgical device the surface treatment includes a plurality of indentation extending into a surface of the surgical device, each of the indentations including a first surface forming a first plane, a second surface forming a second plane, and a third surface forming a third plane. The first, second and third planes are substantially perpendicular to each other. The plurality of indentations form a cluster of indentations visible to an ultrasonic imaging system, and the surface treatment improves visibility of the surgical device by the ultrasonic imaging system. The cluster of indentations may include three indentations in a clover-like formation, each of the indentations being positioned 120 degrees from each other. The cluster of indentations may include two indentations in a minor-like formation positioned 180 degrees from each other.

Also disclosed is a surface treatment formed on the surface of a surgical device. The surface treatment includes a plurality of indentation extending into a surface of the surgical device, each of the indentations include a first surface forming a first plane, a second surface forming a second plane, and a third surface forming a third plane. The first, second and third planes are substantially perpendicular to each other. The plurality of indentations form a pattern along the surface of the surgical device, and the surface treatment is visible to an ultrasonic imaging system. The surface treatment improves visibility of the surgical device by the ultrasonic imaging system. The pattern along the surface of the surgical device may form a plurality of rows. The angle between the first plane and the surface of the surgical device may alternate between rows wherein a first angle is between 10 and 20 degrees and a second angle is between 70 and 80 degrees.

Also disclosed is a surface treatment formed on the surface of a surgical device. The surface treatment includes a first indentation extending into a surface of the surgical device, a second indentation extending into the surface of the surgical device, wherein the first indentation overlaps the second indentation. The first and second indentations include a first surface forming a first plane a second surface forming a second plane, a third surface forming a third plane, a fourth surface forming a fourth plane, a fifth surface forming a fifth plane, a sixth surface forming a sixth plane, and a seventh surface forming a seventh plane. The first, second and third planes are substantially perpendicular to each other and the fourth and fifth and sixth planes are substantially perpendicular to each other. The surface treatment is visible to an ultrasonic imaging system, and the surface treatment improves visibility of the surgical device by the ultrasonic imaging system.

### BRIEF DESCRIPTION OF THE DRAWINGS

FIGS. 1A-1B are perspective views of the positioning assembly according to an embodiment of the present disclosure including a positioning introducer and an outer jacket;
FIG. 2A is an illustration of the positioning assembly of FIG. 1B partially inserted into tissue;
FIG. 2B is an illustration of the positioning introducer removed from the jacket after the jacket is positioned in a target tissue.
FIG. 3A is a perspective view of a microwave energy delivery assembly according to another embodiment of the present disclosure including a microwave energy delivery device and an outer jacket;
FIG 3B is a cross sectional view of the assembled microwave energy delivery assembly of FIG. 3A;
FIG. 4A is an illustration of the microwave energy delivery device being inserted into the jacket positioned in a tissue pathway;
FIG. 4B is an illustration of the energy delivery device assembly positioned in a tissue pathway;
FIGS. 5A-5D are prospective views of various jacket configurations according to an embodiment of the present disclosure.
FIG. 6A is a perspective view of a positioning assembly according to an embodiment of the present disclosure including a surface treatment on the introducer;
FIG. 6B is a perspective view of an ablation apparatus according to an embodiment of the present disclosure include a surface treatment on the outer surface;
FIG. 7A is a cross sectional view of the surface treatment reflecting an ultrasonic signal;
FIG. 7B is a perspective view of a surface treatment reflecting two ultrasonic signals;
FIG. 8A is a perspective view of a surface treatment pattern according to an embodiment of the present disclosure;
FIG. 8B is a perspective view of a repeating surface treatment pattern according to an embodiment of the present disclosure;
FIGS. 9A-9B are views of a surface treatment patterns according to embodiments of the present disclosure; and
FIG. 10 is a surface treatment pattern according to an embodiment of the present disclosure.

### DETAILED DESCRIPTION

Embodiments of the presently disclosed assemblies, systems and methods are described in detail with reference to the drawing figures wherein like reference numerals identify similar or identical elements. As used herein and as is traditional, the term "distal" refers to the portion which is furthest from the user and the term "proximal" refers to the portion that is closest to the user. In addition, terms such as "above", "below", "forward", "rearward", etc. refer to the orientation of the figures or the direction of components and are simply used for convenience of description.

During invasive treatment of diseased areas of tissue in a patient, the insertion and placement of an electrosurgical energy delivery apparatus, such as a microwave antenna assembly, relative to the diseased area of tissue is important for successful treatment. Generally, assemblies described herein allow for placement of a microwave antenna in a target tissue in a two step process. In a first step, a positioning assembly is directly inserted and positioned into target tissue, and in a second step, the positioning introducer is removed from a positioning jacket and replaced with a microwave energy delivery device, the jacket and microwave energy delivery device thereby forming an energy delivery device assembly in the target tissue.

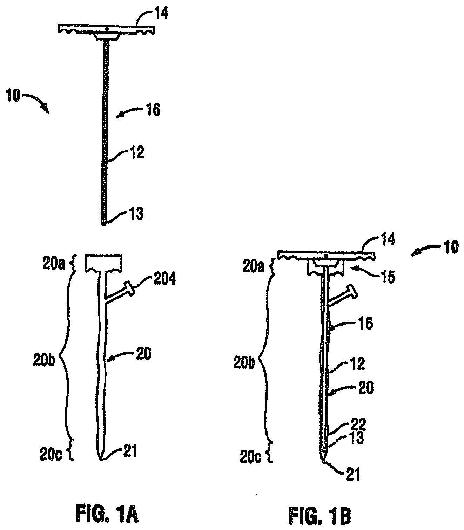

Referring now to FIGS. 1A-1B, a positioning assembly, according to an embodiment of the present disclosure, is shown as 10. The positioning assembly 10 includes a positioning introducer 16 and a jacket 20. The positioning introducer 16 includes a handle 14 that connects to an elongated shaft 12. The elongated shaft 12 includes a tip 13 at a distal end thereof. Jacket 20 includes a receiver portion 20a, a sheath portion 20b, a receptacle tip portion 20c and a fluid outlet 204. Sharpened tip 21 (on the distal end of the receiver portion 20a) is configured to be percutaneously inserted into tissue to define a pathway therethrough.

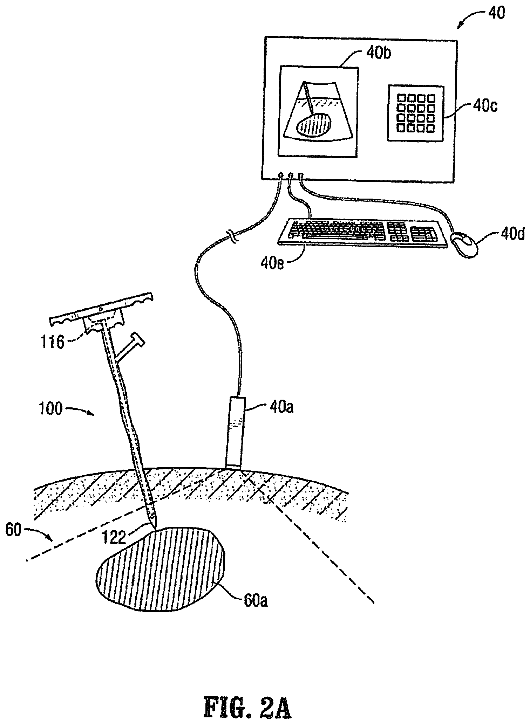

As illustrated in FIG. 2a, the positioning introducer 16 is configured to slideably engage the jacket 20 and forms a percutaneously insertable positioning assembly 10. Receiver portion 20a of the jacket 20 is configured to receive at least a portion of the handle 14 of the positioning introducer 16 thereby forming an assembly handle 15. Assembly handle 15, when grasped by a clinician, enables the clinician to control the positioning assembly 10 during insertion. Sheath portion 20b is configured to slideably engage the elongated shaft 12.

Receptacle tip portion 20c is configured to receive and engage at least a portion of tip 13 thereby forming a structurally rigid tip assembly 22 with the sharpened tip 21 on the distal end of the positioning assembly 10.

Elongated shaft 12 and tip 13 of positioning introducer 16 are configured to produce a highly identifiable image on a suitable imaging system used to aid in the positioning of an ablation device in target tissue. The elongated shaft 12 and tip 13 may be highly identifiable due to one or more materials used in their construction and/or one or more identifiable features incorporated into the design and/or the materials of the positioning introducer 16.

In one embodiment, the elongated shaft 12 and tip 13 of the positioning introducer 16 are readily identifiable by an ultrasonic imaging system 40, as illustrated in FIG. 2A. Ultrasonic imaging system 40 includes an imaging device 40a, such as, for example, a suitable ultrasonic transducer, a display 40b and one or more suitable input devices such as, for example, a keypad 40c, keyboard 40e, a pointing device 40d and/or an external display (not explicitly shown),

As illustrated in FIG 2A, the positioning assembly 100 is percutaneously inserted into patient tissue 60. During insertion, the disposition of the positioning assembly 100 with respect to the target tissue is percutaneously observed on the display 40b of the imaging device 40. The hyperechoic positioning introducer 116 of the positioning assembly 100 is easily identifiable on display 40b. The positioning assembly 100 is guided by a clinician into a desirable position within a portion of the target tissue 60a while the clinician percutaneously observes the advancement of the positioning assembly 100 on the display 40b forming a pathway in tissue.

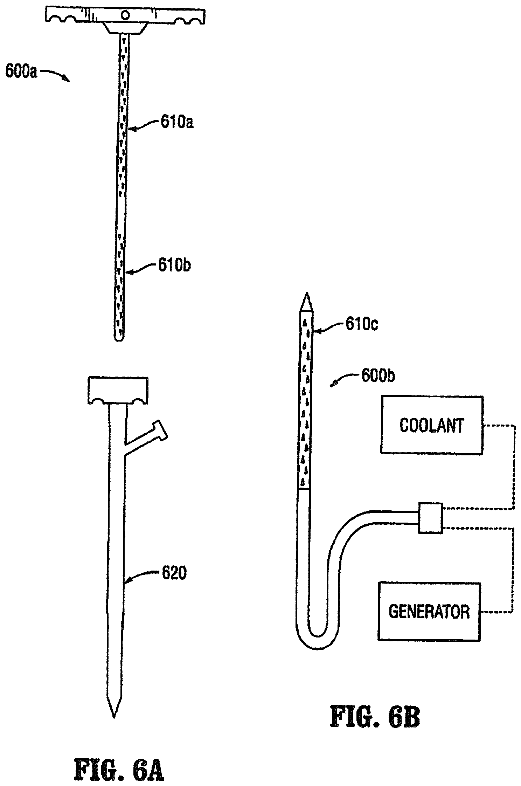

Various echogenic treatments may be applied to the positioning introducer 116 to enhance the ability of the ultrasonic imaging device 40 to replicate the positioning introducer on the display 40b. In one embodiment, the positioning introducer 116 includes a surface dispersion treatment. The surface dispersion treatment may include a dimpled surface or a surface imbedded with particles wherein the surface dispersion treatment creates wide angles of dispersion of the energy transmitted from the imaging device 40a. In another embodiment, the positioning introducer 116 is formed from a composite material that includes particles or fibers bonded within the structure wherein the orientation of the particles or fibers create a wider angle of dispersion of the energy transmitted from the imaging device 40a. Surface treatments that may be applied to the introducer and/or an ablation device are illustrated in FIGS. 6-10 and described hereinbelow.

In yet another embodiment, the positioning introducer 116 includes resonant materials or structures configured to resonate when exposed to energy transmitted from the imagine device 40a. The positioning introducer 116 may include materials, such as crystalline polymers, that absorb energy and resonate when exposed to the energy transmitted from the imaging device 40a. Alternatively, the surface of the positioning introducer 116 may include specific geometries, such as, for example, wall thickness of the positioning introducer 116, gaps defined in a periphery of the positioning introducer 116, a groove or a series of groves defined in a periphery of the positioning introducer 116 and/or fins extending from a periphery of the positioning introducer 116, wherein the specific geometry is configured to resonate at the frequency of the energy transmitted from the imagine device 40a.

The positioning introducer 116 may further include a treatment configured to improve visibility of the positioning introducer by an ultrasonic imaging system and a resonant material that resonates when exposed to energy transmitted from the ultrasonic imaging system. The resonant material may be a crystalline polymer.

In yet another embodiment, a clinician may utilize a Magnetic Resonance Imaging (MRI) device to observe the positioning introducer 116 during the positioning step. The positioning introducer 116, when used with an MRI device, may include one or more non-ferromagnetic materials with very low electrical conductivity, such as, for example, ceramic, titanium and plastic.

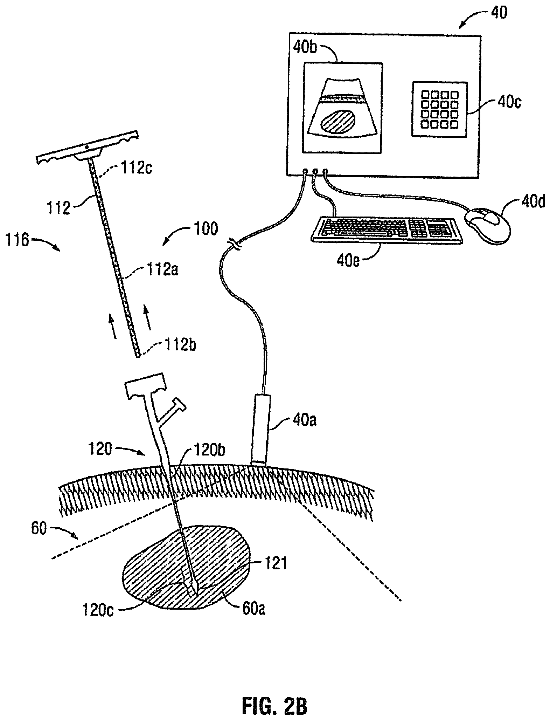

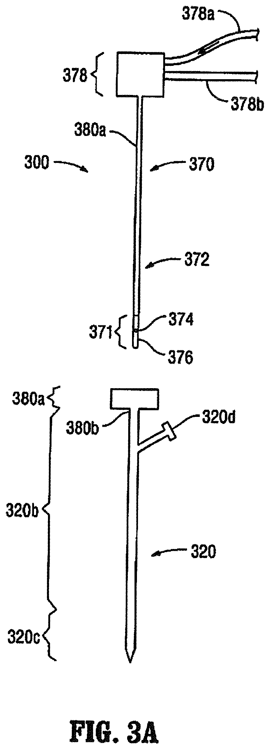

As illustrated in FIG. 2B, after positioning, where the positioning assembly 100 is properly positioned in the target tissue 60a, the positioning introducer 116 is removed from the jacket 120b leaving at least a portion of the jacket 120 in the tissue pathway created during the positioning step. The jacket 120 is further configured to receive a microwave energy delivery device 370 as further described hereinbelow and illustrated in FIGS. 3A-3B.

In one embodiment, at least a portion of the jacket 120 lacks sufficient structural strength to maintain a form and/or a structure in the patient tissue 60 or in the target tissue 60a after the positioning introducer 116 is removed from the jacket 120. For example, during or after removal of the positioning introducer 116 a portion of the jacket 120 may collapse inward and/or upon itself. Collapsing of a portion of the jacket 120, such as the sheath 120b, as illustrated in FIG 2B, may reduce or relieve vacuum created during the removal of the sheath 120b.

In another embodiment the cooling jacket is radially flexible, (e.g. expandable in the radial direction). As such, the positioning introducer 116 of FIGS. 1A-1B may be formed as a smaller gauge than the microwave energy delivery device 370 illustrated in FIGS. 3A-3B. During insertion, the positioning assembly 100 forms a smaller initial puncture site in patient tissue 60 that will typically stretch to accommodate the larger microwave energy delivery device 370 without enlarging or creating a further incision.

Elongated shaft 112 of positioning introducer 116 may provide a passageway for fluids to flow between the distal and proximal ends of the elongated shaft 112. For example, the elongated shaft 112 may form a tip vent hole 112b and a handle vent hole 112c fluidly connected by a lumen 112a. Lumen 112a provides a passageway for fluid (e.g., air, water, saline and/or blood) to flow through the positioning introducer 16 and in or out of the jacket 20 to relieve vacuum or pressure that may be created when the positioning introducer 16 is moved within the jacket 120.

In another embodiment, the outer surface of the elongated shaft 112 may form one or more channels (not explicitly shown) that extend longitudinally between the distal end and the proximal end of the elongated shaft 112. In yet another embodiment, the elongated shaft 112 of the positioning introducer 116 may be formed of a porous material that includes a structure that facilitates the flow of fluid longitudinally between the distal end and the proximal end of the elongated shaft 112.

The sharpened tip 121 may be configured to maintain a form and/or a structure after the removal of the positioning introducer 116 as illustrated in FIG. 2B.

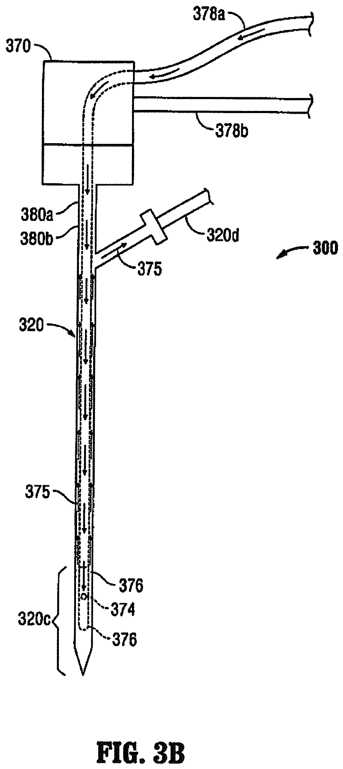

FIG. 3A is a perspective view of the disassembled microwave energy delivery assembly 300 according to an embodiment of the present disclosure. Microwave energy delivery assembly 300 includes a microwave energy delivery device 370 and the jacket 320 of the positioning assembly 10 of FIGS. 1A-1B. The microwave energy delivery device 370 is configured to slideably engage jacket 320 and form a fluid-cooled microwave energy delivery assembly 300 as illustrated in FIG. 3B and described hereinbelow.

Microwave energy delivery device 370 includes an input section 378, a sealing section 380a and an antenna section 372. Input section 378 includes a fluid input port 378a and a power connector 378b. Fluid input port 378a connects to a suitable cooling fluid supply (not explicitly shown) configured to provide cooling fluid to an electrosurgical energy delivery device. A power connector 378b is configured to connect to a microwave energy source such as a microwave generator. Sealing section 380a of the microwave energy delivery device 370 interfaces with the sealing section 380b of the jacket 320 and is configured to form a fluid-tight seal therebetween. Antenna section 372 includes a microwave antenna 371 configured to radiate energy when provided with a microwave energy power signal. A cooling fluid exit port 374 resides in fluid communication with fluid input port 378a. More particularly, fluid supplied to the fluid input port 378a flows through one or more lumens formed within the microwave energy delivery device 370 and exits through the cooling fluid exit port 374. Tip 376 of the microwave energy delivery device 370 is configured to engage receptacle tip 320c of jacket 320.

FIG. 3B is a cross sectional view of the assembled microwave energy delivery assembly of FIG. 3A according to an embodiment of the present disclosure. Microwave energy delivery device 370 slideably engages jacket 320 such that the sealing section 380a and tip 376 of the microwave energy delivery device 370 engage the jacket sealing section 380b and receptacle tip 320c of the jacket 320, respectively, and form a fluid-tight seal therebetween.

In use, the energy delivery device assembly 300 is configured as a fluid-cooled microwave energy delivery device. As illustrated by the flow arrows 375 in FIG. 3B, fluid enters the fluid input port 378a and travels distally through the microwave energy delivery device 370 to the cooling fluid exit port 374. A fluid-tight engagement between the tip 376 and the receptacle tip 320c limits the flow of fluid distally relative to the cooling fluid exit port 374. Fluid that exits the cooling fluid exit port 374 flows proximally through a lumen 376 formed between the outer surface of the microwave energy delivery device 370 and the inner surface of the jacket 320 thereby cooling at least a portion of the sheath portion 320b of the jacket 320. Fluid exits the energy delivery device assembly 300 through the fluid outlet 320d.

The tip 376 of the microwave energy delivery device 370 and the receptacle tip 320c may be any suitable shape provided that tip 376 and receptacle tip 320c mutually engage one another.

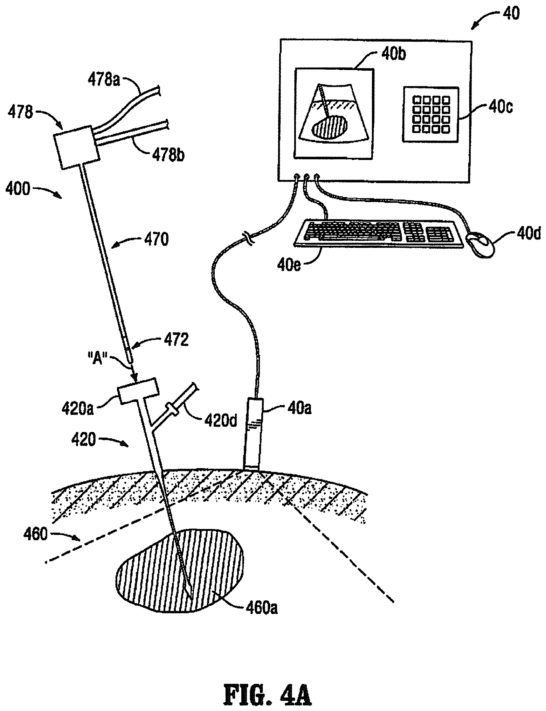

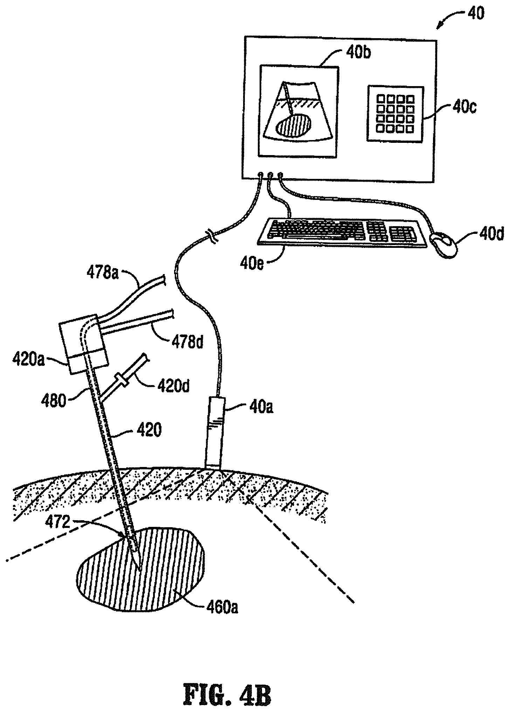

As illustrated in FIGS. 4A and 4B, an energy delivery assembly 400 includes the microwave energy delivery device 470 described similarly hereinabove and illustrated in FIGS. 3A and 3B and the jacket 420 described similarly hereinabove and illustrated in FIGS. 1A-1B and FIGS. 3A-3B and shown as 20 and 320, respectively, The jacket 420 in FIG. 4A and 4B is similar to jacket 320 of the positioning assembly 100 of FIGS 2A-2B (positioned in the pathway in tissue 460 and in the target tissue 460a.) The microwave energy delivery assembly 400 is assembled by inserting the microwave energy delivery device 470 into the jacket 420 as indicated by the arrow "A".

After assembling the microwave energy delivery assembly 400 in the tissue pathway, a fluid supply (not shown) connects to the fluid input port 478a, a fluid drain connects to the fluid outlet 420d and a suitable microwave energy signal source connects to the power connector 478b. Fluid is circulated through the microwave energy delivery assembly 400 in a similar fashion as described above and energy is delivered to the target tissue 460a through the antenna 472 of the microwave energy delivery device 470.

After a suitable amount of energy is delivered to the target tissue 460a, the microwave energy delivery assembly 400 is removed from the tissue pathway. In one embodiment, the assembly 400 is removed by grasping the receiver portion 420a of the jacket 420 and the input section 478 of the microwave energy delivery device 470 and withdrawing the assembly from the patient.

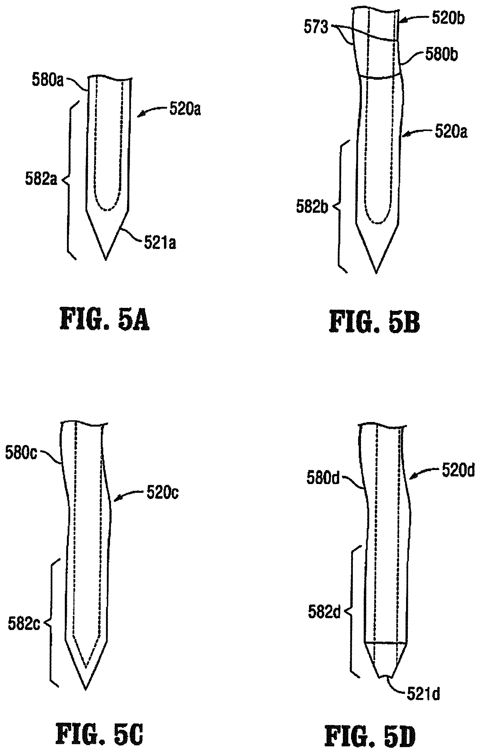

FIGS. 5A-5D are each cross-sectional views of the distal portion of a jacket 520a - 520d according to various embodiments of the present disclosure. In FIG. 5A, jacket 520a includes a semi-rigid sheath 580a and a semi-rigid receptacle tip 582a. The semi-rigid receptacle tip 582a forms a sharpened tip 521a at the distal end that is sufficiently rigid to pierce tissue. In FIG. 5B, jacket 520b includes a flexible sheath 580b and a semi-rigid receptacle tip 582b. Flexible sheath 580b may stretch in diameter and/or length to accommodate the positioning introducer and/or the microwave energy delivery device when inserted into the jacket 520b as described hereinabove. In one embodiment, at least a portion of the receptacle tip 582b forms a portion of the microwave antenna 571b and radiates energy to tissue. In yet another embodiment at least a portion of the sheath 580b includes a microwave energy choke 573 capable of preventing energy from traveling proximally from the antenna.

In FIG. 5C, jacket 520c includes a flexible sheath 580c and a rigid receptacle tip 582c. Jacket 520c is configured to receive a sharpened or pointed tip. In FIG. 5D, jacket 520d includes a flexible sheath 582d and a flexible receptacle tip 582d. A distal tip 521d is configured to receive a positioning introducer and microwave energy delivery device with a sharpened tip. The receptacle tip 582d is configured to form a water-tight seal between the jacket 520d and the introducer (e.g., introducer 16, see FIG. 1) and/or the delivery device (e.g., delivery device 370, see FIG 3A) inserted therewithin.

FIGS. 6-10 are view of surface treatments that may be applied to an introducer and/or an ablation device to improve visibility of the positioning introducer and/or the ablation device by an ultrasonic imaging system. Surface treatments described herein and applied to a surgical device improves the visibility of the device by an ultrasonic imaging system. The various surface treatments may be formed on the surface of a surgical device during an assembly, formed as a step in the manufacturing process and/or etched or otherwise formed on the surface through a secondary treatment process.

As illustrated in FIGS. 6A and 6B, a surface treatment 610a-610b may be applied to an introducer 600a or a surface treatment 610c may be applied to an ablation apparatus 600b. Introducer 600a and ablation apparatus 600b may be used in cooperation with a jacket 620, as described hereinabove. Alternatively, ablation apparatus 600b may be used as a percutaneously inserted device for use without a jacket 620.

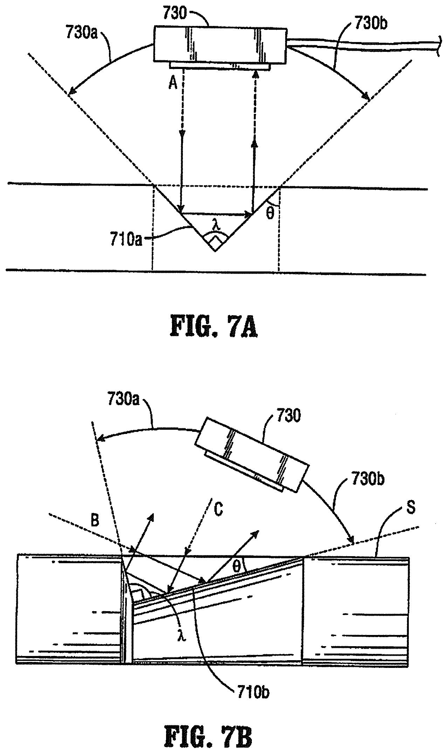

FIG. 7A is a cross sectional view of a surface treatment 710a reflecting an ultrasonic signal A transmitted from and received by an ultrasonic device 730. The geometrical structure of the surface treatments 710a reflects the ultrasonic signal A, transmitted from the ultrasonic device 730, to the ultrasonic device 730. The surface treatment 710a reflects the ultrasonic signal A to the ultrasonic device 730 through a range of motion of the ultrasonic device 730, as illustrated with arrows 730a and 730b. The internal cut angle λ of the surface treatment 710a formed as a right angle directs the reflected ultrasonic signal A along paths that are substantially parallel, as illustrated in FIG. 7A. Decreasing the internal cut angle λ less than 90 degrees (e.g. in a range between 80 and 90 degrees) angles the reflected ultrasonic signal A toward the ultrasonic source 730 while increasing the internal cut angle λ (e.g., in a range between 90 and 100 degrees) angles the reflected ultrasonic signal A away from the ultrasonic source 730. The internal cut angle λ may be varied between an internal cut angle λ between 80 and 90 degrees, an internal cut angle λ between 85 and 95 degrees and/or an internal cut angle λ between 90 and 100 degrees. In FIG. 7A the approach angle θ is approximately 45 degrees on each side of the surface treatment 710a.

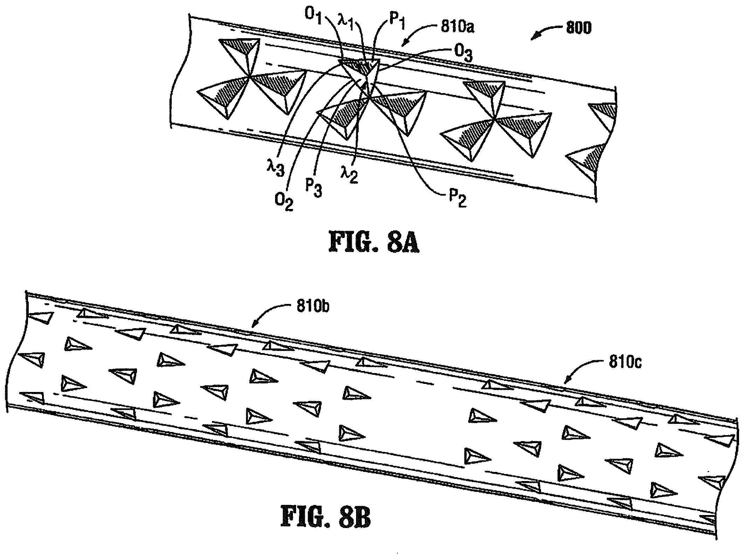

FIG. 7B is a perspective view of a surface treatment 710b reflecting two ultrasonic signals B, C. Varying the approach angle θ of the surface treatment 710b varies the range of motion 730a, 730b of the ultrasonic device 730. As such, the surface treatment 710b with an approach angle θ, as illustrated in FIG. 7B reflects ultrasonic signals that are transmitted from one end of the device (e.g., ultrasonic signal C) while failing to reflect signals from the other end of the device (e.g., ultrasonic signal B). Approach angle θ may vary between 5 - 85 degrees or approach angle θ may vary between 10-20 degrees. In other embodiments, the approach angle θ may vary and/or alternate between 10-20 degrees and 70-80 degrees. Varying and/or alternating the approach angle θ increases the range of motion 730a-730b from about 100 degrees, as illustrated in FIGS. 7A and 7B, to a range of motion between 20 and 160 degrees, as illustrated in FIGS. 8A, 8B, 9A, 9B and 10 and described hereinbelow.

FIG. 8A illustrates a cluster surface treatment 810a formed on a surface 800. The cluster surface treatment 810a includes a plurality of clusters wherein each cluster includes three indentations oriented in a pattern wherein each indentation is rotated 120 degrees with respect to each other (e.g., a clover-like pattern). The clusters forming the cluster surface treatment 810 are arranged along the surface 800 to provide varying reflecting surfaces. The clusters may be fixed in orientation along the longitudinal axis, as illustrated in FIG. 8A, or the clusters orientation may vary with respect to each other around the circumference and/or along the longitudinal length thereof.

Each triangular-shaped indentation include three surfaces forming planes P1, P2, P3 wherein the intersection of each plane forms an internal cut angle λ₁, λ₂, λ₃ and a corresponding approach angles θ₁, θ₂, θ₃ (see also FIGS. 7A-7B described hereinabove). As such, the individual triangular-shaped indentations include three internal cut angle λ₁, λ₂ λ₃ that may vary between 80 and 100 degrees and corresponding approach angles θ₁, θ₂, θ₃ that may vary between 5 and 45 degrees and 45 and 85 degrees (see FIGS. 7A-7B discussed hereinabove).

FIG. 8B shows a perspective view of a surface treatment 810b, 810c formed with an alternative pattern of indentations. The approach angles θ₁, θ₂, θ₃ (not explicitly shown, see FIG. 8A) between rows may alternate between an approach angle θ₁, θ₂, θ₃ of 5 to 45 degrees (e.g. rows one, three, five etc...) and an approach angle θ₁, θ₂, θ₃ of 45 to 85 degrees (e.g., rows two, four, six, etc...). Approach angles θ₁, θ₂, θ₃ may be fixed angle (e.g., alternating between 15 and 75 degrees or alternating between 20 and 70 degrees, etc...) or the approach angle θ₁, θ₂, θ₃ may alternate and vary (alternating between rows and varying between 10-20 degrees and 70 and 80 degrees).

The surface treatment 810a and 810b may include portions without indentations, as illustrated in FIG. 8B. Portions without indentations may indicate a particular section of the device (e.g., an antenna region, choke region or return electrode region). A portion without indentations may also indicate a measurement point such as indicating a fixed distance with respect to an antenna region, choke region or return electrode region.

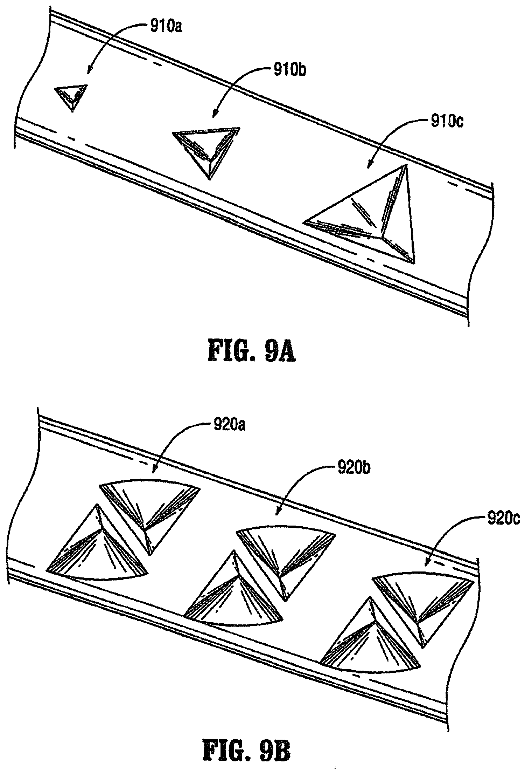

FIGS. 9A illustrates a surface treatment 900 formed with a plurality of indentations 910a, 910b, 910c wherein the indentations vary in orientation, position and size. FIG. 9B illustrates a surface treatment 920 with indentation clusters 920a, 920b, 920c. The indentation clusters 920a, 920b, 920c each include two indentations wherein the indentation in each indentation cluster 920a, 920b, 920c are offset by 180 degrees with respect to each other.

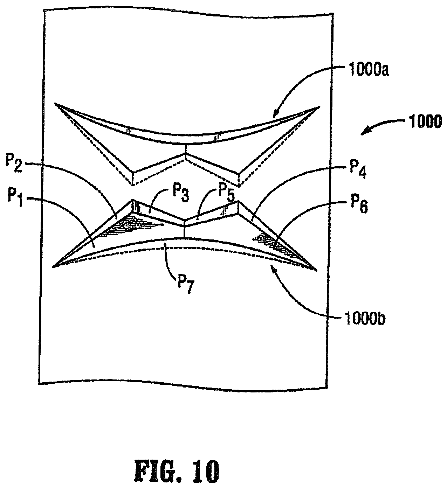

FIG. 10 illustrates a surface treatment 1000 that includes a plurality of clusters 1000a, 1000b wherein each cluster includes two overlapping indentations. The clusters 1000a, 1000b forming the surface treatment 1000 each include seven surfaces that form corresponding planes P1, P2, P3, P4, P5, P6 and P7. The intersection of the planes P1, P2, P3, P4, P5, P6 and P7 form corresponding internal cut angles (not explicitly shown) and corresponding approach angles (not explicitly shown). The planes P1, P2, P3, P4, P5, P6 and P7 provide varying reflecting surfaces that reflect ultrasonic signals transmitted from various angles and orientations. Clusters 1000a, 1000b are illustrated as mirror-images although other suitable arrangement between the clusters 100a, 1000b may be utilized. The orientation between clusters may vary along the circumference and/or varied along the longitudinal length thereof.

Plane P7, which extends between the two overlapping indentations, may be eliminated by forming indentations similarly to the indentations illustrated in FIGS. 7A-9B and described hereinabove.

The assemblies and methods of using the assemblies discussed above are not limited to microwave antennas used for hyperthermic, ablation, and coagulation treatments but may include any number of further microwave antenna applications. Modification of the above-described assemblies and methods for using the same, and variations of aspects of the disclosure that are obvious to those of skill in the art are intended to be within the scope of the claims.
The invention is described by the following numbered paragraphs:-
1. A surface treatment formed on the surface of a surgical device, the surface treatment including:
   an indentation extending into a surface of the surgical device, the indentation including:
      a first surface forming a first plane;
      a second surface forming a second plane, and
      a third surface forming a third plane,
      wherein the first, second and third planes are substantially perpendicular to each other;
   wherein the surface treatment is visible to an ultrasonic imaging system and the surface treatment improves visibility of the surgical device by the ultrasonic imaging system.
2. The surface treatment according to paragraph 1, wherein an angle between the first plane and the surface of the surgical device is between 10 and 20 degrees.
3. The surface treatment according to paragraph 1, wherein an angle between the first plane and the surface of the surgical device is between 70 and 80 degrees.
4. A surface treatment formed on the surface of a surgical device the surface treatment including:
   a plurality of indentations extending into a surface of the surgical device, each of the indentations including:
      a first surface forming a first plane;
      a second surface forming a second plane, and
      a third surface forming a third plane,
      wherein the first, second and third planes are substantially perpendicular to each other;
   wherein the plurality of indentations form a cluster of indentations, and
   wherein the surface treatment is visible to an ultrasonic imaging system and the surface treatment improves visibility of the surgical device by the ultrasonic imaging system.
5. The surface treatment according to paragraph 4, wherein the cluster of indentations include three indentations in a clover-like formation positioned 120 degrees from each other.
6. The surface treatment according to paragraph 4, wherein the cluster of indentations include two indentations in a mirror-like formation positioned 180 degrees from each other.
7. A surface treatment formed on the surface of a surgical device, the surface treatment including:
   a plurality of indentations extending into a surface of the surgical device, each of the indentations including:
      a first surface forming a first plane;
      a second surface forming a second plane, and
      a third surface forming a third plane,
      wherein the first, second and third planes are substantially perpendicular to each other;
   wherein the plurality of indentations form a pattern along the surface of the surgical device and
   wherein the surface treatment is visible to an ultrasonic imaging system and the surface treatment improves visibility of the surgical device by the ultrasonic imaging system.
8. The surface treatment according to paragraph 7, wherein the pattern along the surface of the surgical device forms a plurality of rows.
9. The surface treatment according to paragraph 8, wherein an angle between the first plane and the surface of the surgical device alternates between a first angle and a second angle between rows, wherein the first angle is between 10 and 20 degrees and the second angle is between 70 and 80 degrees.
10. A surface treatment formed on the surface of a surgical device, the surface treatment including:
   a first indentation extending into a surface of the surgical device;
   a second indentation extending into the surface of the surgical device, the first indentation overlapping the second indentation, wherein the first and second indentations include:
      a first surface forming a first plane;
      a second surface forming a second plane;
      a third surface forming a third plane;
      a fourth surface forming a fourth plane;
      a fifth surface forming a fifth plane;
      a sixth surface forming a sixth plane; and
      a seventh surface forming a seventh plane
      wherein the first, second and third planes are substantially perpendicular to each other and the fourth and fifth and sixth planes are substantially perpendicular to each other;
   wherein the surface treatment is visible to an ultrasonic imaging system and the surface treatment improves visibility of the surgical device by the ultrasonic imaging system.

## Claims

1. An ablation device, comprising:
a first indentation and a second indentation, each extending into an outer surface (S) of the ablation device, each indentation including:
a first surface forming a first plane (710a, 710b) extending into the outer surface of the ablation device at a first approach angle θ₁, wherein the approach angle is the angle subtended by the first plane relative to the outer surface (S) of the ablation device;
a second surface forming a second plane; and
a third surface forming a third plane,
wherein the first, the second, and the third planes are substantially perpendicular to each other; and
wherein the first plane of the the second indentation extends into the outer surface of the ablation device at a second approach angle θ₂, different from the first approach angle θ₁ of the first plane of the first indentation.

2. The ablation device according to claim 1, wherein at least one of the first approach angle or the second approach angle is between 5 degrees and 45 degrees.

3. The ablation device according to claim 1, wherein at least one of the first approach angle or the second approach angle is between 45 degrees and 85 degrees.

4. The ablation device according to claim 1, wherein the first indentation and the second indentation are oriented about 180 degrees relative to each other to form a mirror-like cluster.

5. The ablation device according to claim 1, wherein the first indentation differs in size, position and orientation relative to the second indentation.

6. The ablation device according to claim 1, comprising rows of indentations, wherein the approach angles θ₁, θ₂ of the first planes of the indentations alternate between rows.

## Patentansprüche

1. Ablationsvorrichtung, die umfasst:
eine erste Vertiefung und eine zweite Vertiefung, die sich jeweils in eine äußere Fläche (S) der Ablationsvorrichtung erstrecken, wobei jede Vertiefung umfasst:
eine erste Fläche, die eine erste Ebene (710a, 710b) bildet, die sich in die äußere Fläche der Ablationsvorrichtung bei einem ersten Annäherungswinkel θ₁ erstreckt, wobei der Annäherungswinkel der Winkel ist, dem die erste Ebene bezüglich der äußeren Fläche (S) der Ablationsvorrichtung gegenüberliegt;
eine zweite Fläche, die eine zweite Ebene bildet; und
eine dritte Fläche, die eine dritte Ebene bildet,
wobei die erste, die zweite und die dritte Ebene im Wesentlichen zueinander senkrecht sind; und
wobei sich die erste Ebene der zweiten Vertiefung in die äußere Fläche der Ablationsvorrichtung mit einem zweiten Annäherungswinkel θ₂, der von dem ersten Annäherungswinkel θ₁ der ersten Ebene der ersten Vertiefung unterschiedlich ist, erstreckt.

2. Ablationsvorrichtung nach Anspruch 1, wobei mindestens einer des ersten Annäherungswinkels oder des zweiten Annäherungswinkels zwischen 5 Grad und 45 Grad beträgt.

3. Ablationsvorrichtung nach Anspruch 1, wobei mindestens einer des ersten Annäherungswinkels oder des zweiten Annäherungswinkels zwischen 45 Grad und 85 Grad beträgt.

4. Ablationsvorrichtung nach Anspruch 1, wobei die erste Vertiefung und die zweite Vertiefung um etwa 180 Grad zueinander ausgerichtet sind, um eine spiegelbildliche Formation zu bilden.

5. Ablationsvorrichtung nach Anspruch 1, wobei die erste Vertiefung hinsichtlich der Größe, Position und Ausrichtung bezüglich der zweiten Vertiefung unterschiedlich ist.

6. Ablationsvorrichtung nach Anspruch 1, die Reihen von Vertiefungen umfasst, wobei die Annäherungswinkel θ₁, θ₂ der ersten Ebenen der Vertiefungen zwischen Reihen abwechseln

## Revendications

1. Dispositif d'ablation, comprenant :
une première indentation et une seconde indentation, chacune s'étendant dans une surface extérieure (S) du dispositif d'ablation, chaque indentation incluant :
une première surface formant un premier plan (710a, 710b) s'étendant dans la surface extérieure du dispositif d'ablation à un premier angle d'approche θ₁, dans lequel l'angle d'approche est l'angle délimité par le premier plan par rapport à la surface extérieure (S) du dispositif d'ablation ;
une deuxième surface formant un deuxième plan ; et
une troisième surface formant un troisième plan,
dans lequel les premier, deuxième et troisième plans sont sensiblement perpendiculaires les uns aux autres ; et
dans lequel le premier plan de la seconde indentation s'étend dans la surface extérieure du dispositif d'ablation à un second angle d'approche θ₂, différent du premier angle d'approche θ₁ du premier plan de la première indentation.

2. Dispositif d'ablation selon la revendication 1, dans lequel au moins l'un parmi le premier angle d'approche ou le second angle d'approche est entre 5 degrés et 45 degrés.

3. Dispositif d'ablation selon la revendication 1, dans lequel au moins l'un parmi le premier angle d'approche ou le second angle d'approche est entre 45 degrés et 85 degrés.

4. Dispositif d'ablation selon la revendication 1, dans lequel la première indentation et la seconde indentation sont orientées d'environ 180 degrés l'une par rapport à l'autre pour former un groupe semblable à un miroir.

5. Dispositif d'ablation selon la revendication 1, dans lequel la première indentation diffère en taille, en position et en orientation par rapport à la seconde indentation.

6. Dispositif d'ablation selon la revendication 1, comprenant des rangées d'indentations, dans lequel les angles d'approche θ₁, θ₂ des premiers plans des indentations alternent entre des rangées
